(19) Europäisches Patentamt
European Patent Office
Office européen des brevets

(11) Numéro de publication : **0 046 707**
**B1**

(12) # FASCICULE DE BREVET EUROPÉEN

(45) Date de publication du fascicule du brevet : **30.07.86**

(21) Numéro de dépôt : **81401299.3**

(22) Date de dépôt : **13.08.81**

(51) Int. Cl.⁴ : **C 07 C103/49**, C 07 C103/50, C 07 C103/76, C 07 C103/85, C 07 D211/70, C 07 D295/18, A 61 K 31/16, A 61 K 31/395

(54) **Dérivés de l'acide amino-4 butyrique et les médicaments, actifs notamment sur le système nerveux central, en contenant.**

(30) Priorité : **27.08.80 FR 8018609**

(43) Date de publication de la demande :
**03.03.82 Bulletin 82/09**

(45) Mention de la délivrance du brevet :
**30.07.86 Bulletin 86/31**

(84) Etats contractants désignés :
**AT BE CH DE FR GB IT LI LU NL SE**

(56) Documents cités :
**CH-A- 356 121**
**DE-A- 1 927 692**
**GB-A- 732 415**
**Le dossier contient des informations techniques présentées postérieurement au dépôt de la demande et ne figurant pas dans le présent fascicule.**

(73) Titulaire : **SANOFI, société anonyme**
**40, Avenue George V**
**F-75008 Paris (FR)**

(72) Inventeur : **Chambon, Jean-Pierre**
**Chemin des Truquets Route de Montpellier**
**F-34570 Montarnaud (FR)**
Inventeur : **Molimard, Jean-Charles**
**782 rue des Combelles**
**F-34980 St Gely du Fesc (FR)**

(74) Mandataire : **Gillard, Marie-Louise et al**
**Cabinet Beau de Loménie 55, Rue d'Amsterdam**
**F-75008 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne des dérivés de l'acide amino-4 butyrique possédant des propriétés pharmacologiques sur le système nerveux central.

Plus particulièrement, l'invention concerne en tant que produits nouveaux des amides de l'acide amino-4 butyrique répondant à la formule générale :

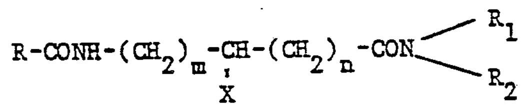

(I)

dans laquelle :

R désigne un groupe alkyle droit ou ramifié ayant de 1 à 7 atomes de carbone ou un groupe cyclopropane ;

m représente un nombre entier de 0 à 3 ;

n représente un nombre entier de 0 à 2, avec la condition que m/n soit égal ou supérieur à 1 ;

X représente l'hydrogène, un groupe alkyle inférieur (1 à 4 carbones), un groupe phénylalkyle dans lequel le groupe phényle est éventuellement substitué ;

$R_1$ et $R_2$ considérés séparément désignent l'hydrogène, un groupe alkyle droit ou ramifié ayant de 1 à 18 atomes de carbone, un groupe aralkyle, un groupe cycloalkyle ou un groupe phényle éventuellement substitué ; ou encore $R_1$ et $R_2$ considérés ensemble avec l'atome d'azote auquel ils sont liés représentent un hétérocycle à 5 ou 6 chaînons comprenant éventuellement un second hétéroatome tel que la pyrrolidine, la morpholine, la pipérazine ou la pyridine et ses dérivés partiellement ou totalement hydrogénés.

Les composés de l'invention montrent des activités intéressantes sur le système nerveux central et peuvent en particulier être utilisés comme sédatifs, tranquillisants hypnotiques et anxiolytiques.

Dans le DE-A-1 927 692, on a décrit des produits chimiques dont la formule diffère de celle des produits (I) par la nature du radical R ; lesdits produits dudit brevet allemand sont indiqués comme ayant une activité neurologique de type anticonvulsivante, sédative ou hypnotique, mais il ne semble pas que lesdits produits réagissent aux tests d'actographie, de potentialisation de la narcose et d'antagonisme des convulsions provoqués par la bicuculine ou l'électrochoc, comme le font les produits de formule (I).

Les composés selon l'invention peuvent s'obtenir selon le schéma réactionnel suivant :

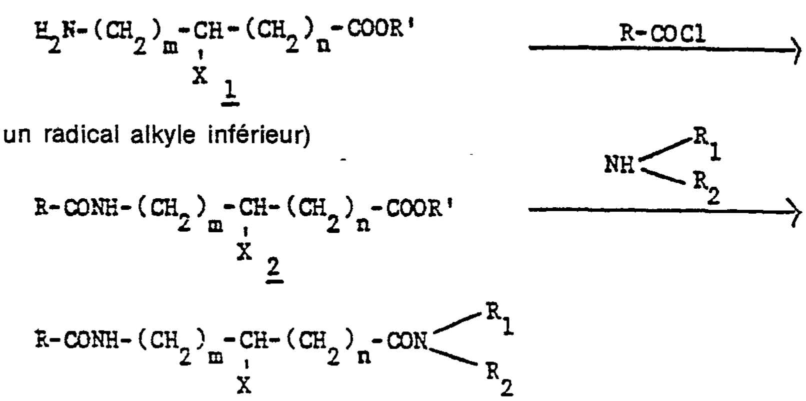


1

(R' étant H ou un radical alkyle inférieur)

2

A partir des aminoacides ou des aminoesters 1 composés connus, on obtient les acides amide 2 par action du chlorure d'acide R—COCl au sein d'un solvant convenable, tel que l'eau, l'éther ou un alcool aliphatique inférieur et en présence d'un accepteur d'hydracide minéral ou organique et, en particulier, la soude ou la triéthylamine.

Les acides ou esters amide 2 sont isolés du mélange réactionnel et, le plus souvent, sont utilisés tels quels sans purification pour l'étape suivante.

L'amidification (réaction avec

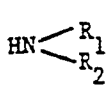

)

peut s'effectuer selon diverses méthodes.

Lorsque R' représente un radical alkyle inférieur, on fait la réaction de l'ester 2 sur l'amine

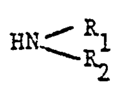

en solution dans un alcool aliphatique inférieur ou en utilisant un excès d'amine comme solvant. On opère en général à une température comprise entre environ 0 et environ 50 °C et le plus souvent à température ambiante. La durée de la réaction peut varier de l'heure à plusieurs jours.

Lorsque R' représente l'hydrogène, on effectue la réaction de l'acide 2 sur l'amine

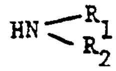

soit en passant par l'intermédiaire de l'ester correspondant (transformation d'acide en un ester d'alcool aliphatique inférieur) soit en utilisant un anhydride mixte formé à partir de l'acide 2. Cet anhydride mixte est formé en faisant réagir l'acide sur le chloroformiate d'éthyle en présence d'un agent alcalin tel que la triéthylamine. On opère dans un solvant convenable tel que le tétrahydrofuranne sans qu'il soit nécessaire d'isoler l'anhydride mixte obtenu. On opère en général à une température entre 0 et 40 °C pendant une durée pouvant varier de 3 à 15 heures environ.

Les exemples suivants, nullement limitatifs, permettent de mieux comprendre la portée de l'invention.

## Exemple 1

Butyl-10 dioxo-4,9 diaza-5,10 tétradécane (CM 40039)

$$R = CH_3\text{—}(CH_2)_2\text{—} ;\ m = n = 1 ;\ X = H ;\ R_1 = R_2 = \text{—}(CH_2)_3CH_3. \qquad (I)$$

a) A une solution de 10,3 g d'acide amino-4 butyrique dans 110 ml d'une solution aqueuse de soude 2N refroidie dans la glace, on ajoute goutte à goutte, sous agitation, 11,7 g de chlorure de butyryle. Après la fin de l'addition, on poursuit l'agitation pendant 4 h.

On lave la solution aqueuse avec du chlorure de méthylène, puis on acidifie la phase aqueuse et on la sature de chlorure de sodium. On extrait avec du chlorure de méthylène, sèche sur sulfate de sodium et évapore à siccité sous vide. Le résidu solide est agité trois fois avec du pentane (300 ml), puis séché sous vide. Poids 9,1 g utilisé tel quel pour l'opération suivante.

b) On dissout l'acide obtenu ci-dessus (9,1 g) dans 200 ml de tétrahydrofuranne sec. On ajoute 6,06 g de triéthylamine et 6,51 g de chloroformiate d'éthyle en maintenant la température inférieure ou égale à 5 °C. On laisse 1 h sous agitation, puis on ajoute goutte à goutte 7,74 g de dibutylamine. On laisse une nuit sous agitation à température ambiante.

On filtre l'insoluble et évapore le solvant à siccité. On reprend le résidu dans l'éther et lave la solution avec une solution diluée d'acide chlorhydrique, puis avec une solution diluée de soude et, enfin, avec une solution aqueuse saturée de chlorure de sodium. On sèche la solution éthérée sur sulfate de sodium, évapore le solvant à siccité et distille le résidu sous vide. On obtient 6,2 g d'un liquide jaune pâle. E/0,01 mm : 170 °C.

## Exemple 2

On opère comme dans l'exemple 1, mais en faisant varier l'amine utilisée au paragraphe b).

On obtient ainsi les produits (I) : $R = CH_3(CH_2)_2$, m = n = 1, X = H, figurant dans le tableau I ci-après.

## Exemple 3

On opère comme dans l'exemple 1, mais en faisant varier, d'une part, le chlorure d'acide utilisé dans la première étape et, éventuellement, d'autre part, l'amine employée dans la seconde étape.

On obtient ainsi les produits (I) rassemblés dans le tableau II ci-après.

## Exemple 4

Butyl-5 éthyl-12 méthyl-13 dioxo-6,11 diaza-5,10 pentadécane (CM 40195)

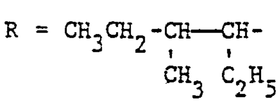

$; m = n = 1; X = H; R_1 = R_2 = -(CH_2)_3CH_3.$ (I)

a) A 9,18 g du chlorhydrate de l'ester benzylique de l'acide amino-4 butyrique dissous dans 100 ml de tétrahydrofuranne, on ajoute 8,08 g de triéthylamine. On refroidit par un bain de glace, puis on ajoute goutte à goutte 6,5 g de chlorure de l'acide éthyl-2 méthyl-3 pentanoïque. On laisse une nuit sous agitation à température ambiante, puis on filtre le mélange réactionnel et évapore le solvant à siccité.

On reprend le résidu dans l'acétate d'éthyle, lave avec de l'eau puis avec une solution diluée de soude, à nouveau avec de l'eau, puis avec une solution diluée d'acide chlorhydrique et, finalement, avec

une solution saturée de chlorure de sodium. On sèche la solution sur sulfate de sodium et évapore le solvant à siccité sous vide.

On obtient ainsi 11 g d'ester benzylique de l'acide éthyl-7 méthyl-8 oxo-6 aza-5 décanoïque-1. On dissout cet ester (11 g) dans 150 ml d'éthanol à 96 et hydrogène à pression atmosphérique en présence de 1 g de palladium sur charbon à 10 % de palladium. Après la fin de réaction, on filtre le catalyseur et évapore à sec sous vide. Au résidu repris dans l'éther anhydre (100 ml), on ajoute 6 g de dicyclohexylamine et laisse une nuit à 0 °C. On essore le sel formé et lave avec de l'éther. Poids 7,8 g.

Le sel ainsi obtenu est dissous dans 100 ml d'eau. On refroidit la solution dans la glace et acidifie par l'acide chlorhydrique concentré jusqu'à pH = 1,5. On sature la solution de chlorure de sodium et extrait avec de l'acétate d'éthyle. On lave trois fois la solution organique avec une solution saturée de chlorure de sodium, sèche sur sulfate de sodium et évapore à siccité.

On obtient ainsi 3,9 g de l'acide éthyl-7 méthyl-8 oxo-6 aza-5 décanoïque-1.

b) Suivant la technique de l'exemple 1 b), on fait réagir sur cet acide la dibutylamine. De la même façon, on obtient CM 40195 sous forme d'une huile ; E.0,01 mm : 200 °C.

## Exemple 5

Dioxo-4,12 diaza-5,13 heptadécane (CM 40387)

$$R = CH_3CH_2CH_2— ; m = 3 ; n = 2 ; X = H ; R_1 = H ; R_2 = (CH_2)_3CH_3. \quad (I)$$

a) A une solution refroidie dans la glace de 5,2 g d'acide amino-7 heptanoïque dans 80 ml de soude 4N, on ajoute lentement sous agitation 4,8 g de chlorure de butyryle. On poursuit l'agitation pendant 4 h, puis on acidifie jusqu'à pH = 2 par l'acide chlorhydrique. On extrait avec de l'acétate d'éthyle, sèche la solution sur sulfate de sodium et évapore le solvant à siccité sous vide.

On obtient une huile (4,2 g) qui cristallise. On recristallise dans l'hexane ; Fk : 68 °C.

b) A la solution de 2,3 g de l'acide obtenu précédemment dans 30 ml de tétrahydrofuranne sec, on ajoute 1,1 g de triéthylamine, puis 1,2 g de chloroformiate d'éthyle. On laisse 2 h sous agitation, puis additionne lentement la solution de 0,85 g de butylamine dans 5 ml de tétrahydrofuranne. On laisse sous agitation pendant 15 h à température ambiante, puis on ajoute de l'eau et extrait avec de l'acétate d'éthyle. On lave la solution organique avec un solution de carbonate de sodium, sèche sur sulfate de sodium et évapore le solvant à siccité. Le résidu est recristallisé dans l'acétonitrile ; Fk : 132°C.

## Exemple 6

En opérant comme dans l'exemple 5 à partir de différents aminoacides 1 et en faisant varier les réactifs R—COCl et

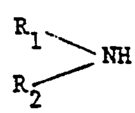

on obtient les produits (I) réunis dans le tableau III ci-après.

## Exemple 7

Dioxo-2,7 diaza-1,6 décane (CM 40401)

$$R = CH_3(CH_2)_2— ; m = n = 1 ; X = H ; R_1 = R_2 = H. \quad (I)$$

a) A une solution de 17 g d'acide oxo-6 aza-5 nonanoïque-1 (obtenu selon l'exemple 1 a)) dans 500 ml d'éthanol absolu, on ajoute 10 ml d'acide sulfurique concentré et agite pendant 4 jours à température ambiante. On évapore le solvant à 30 °C sous vide et reprend le résidu dans l'eau glacée. On neutralise par addition de bicarbonate de sodium, puis on extrait avec du chlorure de méthylène. On sèche sur sulfate de sodium et évapore à siccité.

On obtient 16 g de l'ester éthylique attendu.

b) On introduit les 16 g d'ester éthylique obtenu ci-dessus dans 300 ml d'une solution à 16 % d'ammoniac dans le méthanol. On agite pendant 5 jours à température ambiante. On évapore le solvant à siccité et reprend le résidu par de l'éther. On essore le solide et lave avec de l'acétonitrile.

On obtient des cristaux incolores (10 g). Fk : 135 °C (acétonitrile).

Exemple 8

(Diméthoxy-3,4 benzyl)-6 dioxo-4,9 diaza-5,10 tétradécane (CM 40187)

R = $CH_3CH_2CH_2$-; m = 0; n = 2; X = $-CH_2-$ 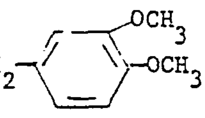 ; $R_1$ = H; (I)

$R_2 = -(CH_2)_3CH_3$.

On opère comme dans l'exemple 7, en remplaçant dans la première réaction le chlorure de chloro-4 benzoyle par une quantité équivalente de chlorure de butyryle.

De la même façon, on obtient le composé attendu sous forme d'un solide incolore ; Fk : 152 °C.

Les produits obtenus selon l'invention ont été soumis à divers essais concernant leur activité pharmacologique et, en particulier, leur action sur le système nerveux central.

A) Activité pharmacologique

1) Effet sédatif et hypnotique.

a) Etude de l'actographie.

La mesure de l'actographie est effectuée chez la souris 45 min. après l'administration du produit. On opère sur des lots de 12 animaux, chacun d'eux étant isolé pendant 10 min. avant la mesure. Le comptage des scores est effectué par coupure de deux faisceaux lumineux perpendiculaires.

Dans le tableau IV ci-après sont rassemblés les résultats obtenus avec divers produits de l'invention administrés à la dose de 500 mg/kg par voie orale. Les résultats sont exprimés en pourcentage de variation des scores obtenus par rapport à des animaux témoins non traités.

On peut constater que les produits se répartissent en deux groupes :

ceux provoquant de l'hypomotilité tels que 40217, 40039, 40271, 40272, 40319 ; dans le cas où les animaux traités présentent une perte du réflexe de retournement, ce qui traduit l'effet de narcose propre du produit, on a noté PRR ;

ceux provoquant de l'hypermotilité, tels que 40142, 40398, 40397, 40404, 40253, 40355, 40209 ; pour préciser les résultats obtenus, l'étude pour deux produits a été reprise en effet-dose suivant le même protocole.

Les résultats obtenus figurent dans le tableau V ci-après.

b) Potentialisation de la narcose au pentobarbital.

Les produits à étudier ont été administrés per os chez la souris à la dose de 500 mg/kg, 1 h avant le pentobarbital injecté par voie intrapéritonéale à raison de 20 mg/kg.

On détermine le pourcentage des animaux ayant perdu le réflexe de retournement. Les résultats sont exprimés en pourcentage ou, dans quelques cas, en dose efficace 50 (DE 50) ou dose provoquant la narcose chez 50 % des animaux traités.

Les résultats figurent dans le tableau VI ci-après.

c) Etude électroencéphalographique.

Pour mieux comprendre l'activité hypnotique des produits selon l'invention, une étude électroencéphalographique a été effectuée sur l'un deux, à savoir CM 40039.

Le 40039 est étudié à la dose de 350 mg/kg p. o. chez trois rats. Après une période d'habituation de 10 jours (période éclairée de 8 h à 20 h et période obscure de 20 h à 8 h), les animaux sont enregistrés pendant 5 jours avec le solvant (gomme arabique à 10 %), puis enregistrés pendant 4 jours pendant lesquels ils reçoivent le produit à 9 h chaque matin. Après cette administration chronique, les animaux sont enregistrés les 5 jours suivants (période de contrôle).

Résultats :

Les animaux sont enregistrés 24 h sur 24. L'étude statistique porte sur des tranches de 24 h. On fait une analyse globale sur les trois rats en cumulant les 15 journées témoins, les 12 journées de traitement, les 15 journées de contrôle. Dans chaque tranche horaire de 24 h, on évalue le temps d'éveil (EV), de sommeil lent (SL), de sommeil paradoxal (SP), de sommeil total (ST), ainsi que le rapport (en %) sommeil paradoxal/sommeil total (SP/ST) (voir tableau VII ci-après).

Pendant toute la période d'étude, les enregistrements n'ont pas révélé de modifications morphologiques du tracé encéphalographique.

Le produit provoque une diminution significative de l'éveil (— 8,1 %) corrélée à une augmentation significative du sommeil lent (+ 6,6 % ).

Cet effet persiste pendant la phase de contrôle.

2) Effet anticonvulsivant.

L'effet anticonvulsivant a été déterminé vis-à-vis des crises convulsives provoquées par l'électrochoc ou par la bicuculline.

L'électrochoc (12,5 V pendant 0,5 s) est effectué chez la souris 60 min après l'administration du produit par voie orale.

La bicuculline est administrée à la souris par voie intraveineuse à raison de 1 mg/kg, 60 min après le produit à étudier donné per os. On note l'effet protecteur obtenu vis-à-vis des crises toniques.

En opérant sur divers lots d'animaux avec des doses différentes du produit à étudier, on peut déterminer la dose efficace médiane (DE 50).

Les résultats sont rassemblés dans le tableau VIII ci-après et montrent les propriétés anticonvulsivantes nettes des produits étudiés.

B) Etude biochimique

a) Effet sur le taux de GABA (acide amino-4 butyrique).

Le 40039 a été administré 30 min avant le sacrifice chez la souris. Le taux de GABA a été évalué sur le cerveau entier (lots de 6 animaux) (voir tableau IX ci-après).

Le 40039 provoque une rapide augmentation du taux de GABA dans le cerveau total chez la souris.

b) Effet sur la dopaminergie centrale.

L'effet des produits sur l'activité dopaminergique centrale a été étudié par la mesure de l'accumulation de l'acide homovanillique sur une période de 24 h après l'administration des produits chez la souris.

Le taux d'acide homovanillique (HVA) est évalué dans le cerveau entier, les animaux reçoivent une injection de probenecid (200 mg/kg i.p.) 1 h 30 min avant le sacrifice (lot de 10 animaux).

Les deux produits provoquent des effets voisins sur le taux de HVA, en particulier, ils provoquent une élévation du taux 4 h après leur administration et une forte baisse 24 h après leur administration (voir tableau X ci-après).

c) Toxicité aiguë.

Les produits à étudier sont administrés par voie orale aux doses de 500 et 1 000 mg/kg à des lots de souris. On observe les souris pendant 24 h et note la mortalité.

Les résultats exprimés en pourcentage de mortalité figurent dans le tableau XI ci-après.

Ils indiquent qu'à la dose de 500 mg/kg, tous les produits étudiés n'ont montré aucun signe de toxicité aiguë. A 1 000 mg/kg, dose très élevée, quelques produits présentent une toxité de 100 %, mais, dans la plupart des cas, la toxicité aiguë reste faible ou nulle.

Les essais ainsi effectués montrent que les produits selon l'invention présentent un ensemble de propriétés pharmacologiques intéressantes et une faible toxicité. Par suite, ils peuvent être utilisés en thérapeutique humaine, notamment pour le traitement des affections neurologiques et psychiques.

En particulier, les produits selon l'invention peuvent être utilisés pour le traitement des troubles de l'humeur ou du comportement : nervosisme, irritabilité ainsi que pour les traitements des états anxieux et les insomnies.

Ces produits peuvent être administrés par voie orale ou par voie injectable. Les compositions pharmaceutiques peuvent être solides ou liquides et se présenter, par exemple, sous forme de comprimés, gélules, granulés, suppositoires ou préparations injectables.

La posologie peut varier dans de larges proportions, en particulier suivant le type et la gravité de l'affection à traiter et suivant le mode d'administration. Le plus souvent chez l'adulte, par voie orale, elle est comprise entre 0,100 et 1 g par jour, éventuellement répartie en plusieurs prises.

A titre d'exemples de compositions pharmaceutiques, on peut citer les préparations suivantes :

Gélules

CM 40039 à 100 mg

| | |
|---|---|
| CM 40039 | 100 mg |
| Aerosil | 0,5 mg |
| Stéarate de magnésium | 1,5 mg |
| Amidon STA RX 1500 | 48 mg |
| | 150 mg |

Comprimés

CM 40142 à 200 mg

| | |
|---|---|
| CM 40142 | 200 mg |
| Cellulose microcristalline | 100 mg |
| Lactose | 197 mg |
| Stéarate de magnésium | 3 mg |
| | 500 mg |

Tableau I

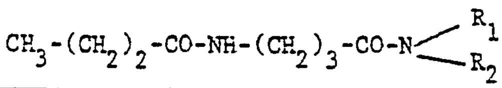

| Numéro de code | $R_1$ | $R_2$ | Point de fusion (°C) (solvant de cristallisation) ou point d'ébullition [°C (pression)] |
|---|---|---|---|
| 40 142 | H | $-(CH_2)_3CH_3$ | 120,5 (acétonitrile) |
| 40 205 | H | $-(CH_2)_7-CH_3$ | 118 (acétonitrile) |
| 40 206 | 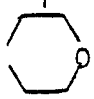 | | 84 (précipité et lavé à l'éther) |
| 40 207 | H | 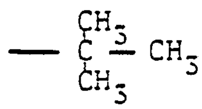 | 102 ( " ) |
| 40 208 | 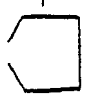 | | 62 ( " ) |
| 40 209 | H | 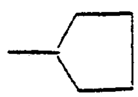 | 134 ( " ) |
| 40 210 | H | 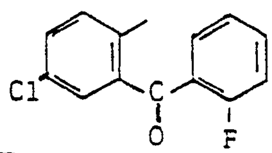 | 122 (isopropanol) |
| 40 211 | 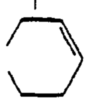 | | ~ 50°C (décomposition) |
| 40 216 | 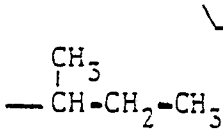 | 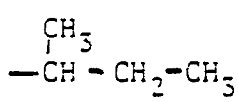 | E. : 185-190 (0,01 mm) |
| 40 217 | $-(CH_2)_2-CH_3$ | $-(CH_2)_2-CH_3$ | E. : 190-194 (0,01 mm) |
| 40 218 | H | $-C_{18}H_{37}$ | 124 (acétonitrile) |
| 40 219 | 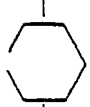 | | 60 (éther anhydre) |
| 40 316 | H | 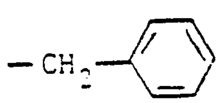 | 132 (acétonitrile) |
| 40 396 | H | $-CH_3$ | 100 (acétonitrile) |
| 40 398 | H | $-(CH_2)_2CH_3$ | 130 (acétonitrile) |

Tableau I (suite)

| Numéro de code | $R_1$ | $R_2$ | Point de fusion (°C) (solvant de cristallisation) ou point d'ébullition [°C (pression)] |
|---|---|---|---|
| 40 463 | H | $-CH_2CH_3$ | 124 (acétonitrile) |
| 40 466 | H | 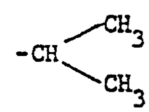 | 124 (acétonitrile) |
| 40 521 | H | $-(CH_2)_4CH_3$ | 110 (acétonitrile) |
| 40 947 | H | $-(CH_2)_5-CH_3$ | 110 (acétonitrile) |
| 40 984 | $-(CH_2)_4-CH_3$ | $-(CH_2)_4-CH_3$ | Huile (chromatographié) |
| 40 987 | H | 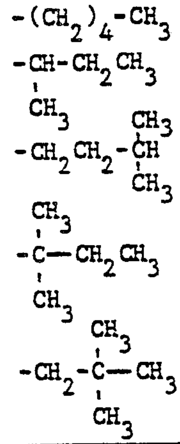 | 97 (acétonitrile) |
| 40 988 | H | | 108 (acétonitrile) |
| 40 989 | H | | 72 (acétonitrile) |
| 40 990 | H | | 70 (acétonitrile) |

Tableau II

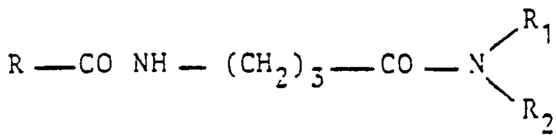

| Numéro de code | R | $R_1$ | $R_2$ | Point de fusion (°C) Point d'ébullition [°C (pression)] |
|---|---|---|---|---|
| 40 254 | 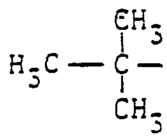 | H | $(CH_2)_3-CH_3$ | 88 (éther) |
| 40 272 | 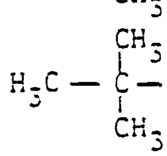 | $-(CH_2)_3CH_3$ | $-(CH_2)_3-CH_3$ | E. : 165-167 (0,01 mm) |
| 40 273 | $CH_3-(CH_2)_6-$ | H | $-(CH_2)_3-CH_3$ | 118 (acétonitrile) |
| 40 274 | $CH_3-(CH_2)_6$ | $-(CH_2)_3CH_3$ | $-(CH_2)_3CH_3$ | E. : 210-215 (0,01 mm) |
| 40 417 | 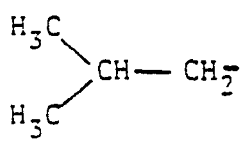 | H | $-(CH_2)_3-CH_3$ | 110 (acétate d'éthyle) |
| 40 418 | 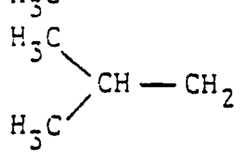 | H | $-(CH_2)_2-CH_3$ | 122 (acétonitrile) |
| 40 440 | 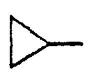 | H | $-(CH_2)_2-CH_3$ | 141 (acétonitrile) |
| 40 443 | 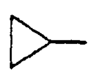 | H | $-(CH_2)_3-CH_3$ | 145 (acétonitrile) |
| 40 462 | 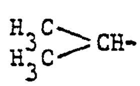 | $-(CH_2)_3-CH_3$ | $-(CH_2)_3-CH_3$ | E. : 184 (0,01 mm) |
| 40 467 | 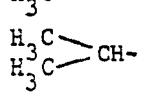 | H | $-(CH_2)_3-CH_3$ | 98 (acétonitrile) |
| 40 885 | 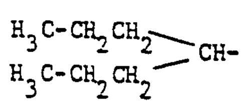 | H | $-(CH_2)_3CH_3$ | 128 (acétonitrile) |

Tableau III

| Numéro de code | R | m | n | X | $R_1$ | $R_2$ | Point de fusion (°C) point d'ébullition [°C (pression)] |
|---|---|---|---|---|---|---|---|
| 40 215 | $CH_3$ — | 2 | 2 | H | $-(CH_2)_3-CH_3$ | $-(CH_2)_3CH_3$ | 69 (éther-hexane) |
| 40 253 | $CH_3-(CH_2)_2-$ | 1 | 0 | H | H | $-(CH_2)_3CH_3$ | 170 (éther) |
| 40 271 | $CH_3-(CH_2)_2-$ | 1 | 0 | H | $-(CH_2)_3CH_3$ | $-(CH_2)_3CH_3$ | E. : 165-7 (0,01 mm) |
| 40 318 | 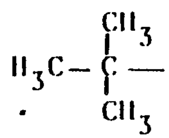 | 1 | 0 | H | H | $-(CH_2)_3CH_3$ | 74 (précipité) |
| 40 319 | 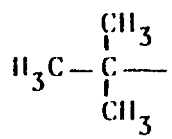 | 1 | 0 | H | $-(CH_2)_3CH_3$ | $-(CH_2)_3CH_3$ | E. : 136-140 (0,01 mm) |
| 40 386 | 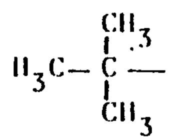 | 3 | 2 | H | H | $-(CH_2)_3CH_3$ | 82 (éther isopylique-benzène) |
| 40 395 | $CH_3-(CH_2)_2$ | 0 | 1 | $-CH_3$ | H | $-(CH_2)_3CH_3$ | 154 (acétonitrile) |
| 40 397 | $CH_3-(CH_2)_2$ | 0 | 2 | $-CH_3$ | H | $-(CH_2)_3CH_3$ | 110 (acétonitrile) |
| 40 404 | $CH_3-(CH_2)_2$ | 2 | 0 | $-CH_3$ | H | $-(CH_2)_3CH_3$ | 100 (acétonitrile) |

Tableau IV

| N° Produits | Mesure de l'actographie (p. cent scores/témoins) |
|---|---|
| 40 217 | - 37 * |
| 40 039 | PRR |
| 40 206 | - 8 |
| 40 271 | - 53 ** |
| 40 401 | - 20 |
| 40 398 | + 54 ** |
| 40 142 | + 50 ** |
| 40 316 | + 16 |
| 40 395 | - 35 |
| 40 397 | + 91 ** |
| 40 404 | + 52 ** |
| 40 272 | - 47 ** |
| 40 319 | - 51 ** |
| 40 253 | + 33 * |
| 40 355 | + 62 ** |
| 40 209 | + 36 * |
| 40 254 | - 2 |
| 40 386 | - 57 ** |
| 40 417 | + 56 ** |
| 40 418 | + 93 ** |
| 40 443 | + 78 ** |
| 40 463 | + 41 ** |
| 40 466 | + 97 ** |
| 40 467 | + 115 ** |
| 40 521 | + 101 ** |
| 40 947 | + 37 * |

$* p \leq 0{,}05$ $** p \leq 0{,}01$

Tableau V

Effet dose du 40039 et du 40142 sur l'actographie.

| CM 40 039 | | | | |
|---|---|---|---|---|
| Dose (mg/kg p. os) | 20 | 40 | 80 | 160 |
| p. cent scores/témoins | - 30 x | - 38 xx | - 37 xx | - 28 |
| x $p \leqslant 0,05$ xx $p \leqslant 0,01$ | | | | |
| CM 40 142 | | | | |
| Dose (mg/kg p. os) | 100 | 200 | 300 | 400 |
| p. cent scores/témoins | + 1 | + 31 | + 38 xx | + 50 xx |
| xx $p \leqslant 0,01$ | | | | |

Tableau VI

| N° Produits | Pourcentage d'animaux en narcose à 500 mg/kg p. os ou dose (p. os) provoquant 50 % d'induction de narcose (DE 50) |
|---|---|
| 40 217 | 0 |
| 40 039 | DE 50 = 200 |
| 40 216 | 100 |
| 40 206 | 0 |
| 40 271 | 100 |
| 40 401 | 0 |
| 40 398 | DE 50 = 350 |
| 40 142 | DE 50 = 297 |
| 40 316 | 70 |
| 40 395 | 70 |
| 40 397 | 100 |
| 40 404 | 40 |
| 40 272 | 72 |
| 40 319 | DE 50 = 175 |
| 40 253 | 50 |
| 40 355 | 40 |
| 40 254 | 50 |
| 40 318 | 90 |
| 40 443 | 40 |
| 40 462 | 30 |
| 40 466 | 30 |
| 40 984 | 60 |
| 40 989 | 50 |

Tableau VII

Analyse du tracé encéphalographique par tranche horaire de 24 h.

| | Témoins | Traités 40039 (325 mg/kg) | Contrôle |
|---|---|---|---|
| EV | 44,2 ± 3,3 | 40,6 ± 3 XXX<br>- 8,1 | 40,3 ± 3,6 XXX<br>- 8,8 |
| SL | 48,3 ± 2,5 | 51,5 ± 2 XXX<br>+ 6,6 | 51 ± 3 XX<br>+ 5,6 |
| SP | 7,6 ± 1,4 | 8,1 ± 1,4 n.s.<br>+ 6,6 | 8,6 ± 1,7 X<br>+ 13,2 |
| ST | 55,9 ± 3 | 59,5 ± 2,6 XXX<br>+ 6,4 | 59,6 ± 3,9 XXX<br>+ 6,6 |
| SP/ST | 12,8 ± 2,1 | 13,2 ± 1,9 n.s.<br>+ 3,1 | 13,5 ± 1,8 n.s.<br>+ 5,5 |

X $p \leqslant 0,1$ XX $p \leqslant 0,05$ XXX $p \leqslant 0,01$

Tableau VIII

| N° Produits | Dose efficace médiane de protection des crises toniques (DE 50) (mg/kg p. os) | |
|---|---|---|
| | Bicuculline | Electrochoc |
| 40 039 | 300 | 250 |
| 40 142 | 325 | 150 |
| 40 253 | < 500 | 500 |
| 40 254 | <500 | - |
| 40 271 | <500 | 500 |
| 40 379 | 450 | - |
| 40 418 | 300 | - |
| 40 462 | 150 | 500 |
| 40 463 | 380 | - |
| 40 467 | 150 | < 500 |
| 40 521 | 250 | 180 |
| 40 947 | 250 | 400 |

Tableau IX

| | Témoins | Traités 40039 500 mg/kg p. os | p.cent/témoins |
|---|---|---|---|
| Taux de GABA en µg/g cerveau | 280 ± 8 | 321 ± 12 * | 14,6 |
| * $p \leq 0{,}05$ | | | |

Tableau X

| Taux de HVA en ng/g | | | | | |
|---|---|---|---|---|---|
| | 0 | 2 h | 4 h | 6 h | 24 h |
| 40 039<br>500 mg/kg p.os | 658±40 | 798±34<br>+ 21%x | 894±37<br>+ 36%xx | 659±29<br>0% | 441±19<br>- 33%xx |
| 40 142<br>500 mg/kg p.os | 521±29 | 379±11<br>- 27%xx | 793±52<br>+ 52%xx | 571±23<br>+ 9 | 290±16<br>- 44 xx |
| x p ⩽ 0,05 xx p ⩽ 0,01 | | | | | |

Tableau XI

| N° Produit | p. cent de mortalité | |
|---|---|---|
| | à 500 mg/kg p. os | à 1000 mg/kg p. os |
| 40 039 | 0 | 100 |
| 40 142 | 0 | 0 |
| 40 206 | 0 | 0 |
| 40 209 | 0 | 20 |
| 40 216 | 0 | 100 |
| 40 217 | 0 | 0 |
| 40 253 | 0 | 0 |
| 40 254 | 0 | 20 |
| 40 271 | 0 | 100 |
| 40 272 | 0 | 0 |
| 40 316 | 0 | 0 |
| 40 319 | 0 | 0 |
| 40 355 | 0 | 0 |
| 40 395 | 0 | 20 |
| 40 397 | 0 | 20 |
| 40 398 | 0 | 0 |
| 40 401 | 0 | 0 |
| 40 404 | 0 | 0 |

Tableau XI (suite)

| N° Produit | p. cent de mortalité | |
|---|---|---|
| | à 500 mg/kg p. os | à 1000 mg/kg p. os |
| 40 417 | 0 | 20 |
| 40 418 | 0 | 0 |
| 40 443 | 0 | 0 |
| 40 462 | 0 | 0 |
| 40 463 | 0 | 0 |
| 40 466 | 0 | 0 |
| 40 467 | 0 | 0 |
| 40 581 | 0 | 0 |
| 40 947 | 0 | 0 |
| 40 984 | 0 | 100 |
| 40 989 | 0 | 80 |

**Revendications** (pour les Etats contractants : BE, CH, DE, FR, GB, IT, LI, LU, NL, SE)

1. Amides de l'acide amino-4 butyrique répondant à la formule générale :

$$R\text{-}CONH\text{-}(CH_2)_m\text{-}\underset{X}{CH}\text{-}(CH_2)_n\text{-}CON\begin{matrix} R_1 \\ R_2 \end{matrix} \qquad (I)$$

dans laquelle

R désigne un groupe alkyle droit ou ramifié ayant de 1 à 7 atomes de carbone ou un groupe cycloalkyle ;

m représente un nombre entier de 0 à 3 ;

n représente un nombre entier de 0 à 2, avec la condition que m + n soit égal ou supérieur à 1 ;

X représente l'hydrogène, un groupe alkyle inférieur (1 à 4 carbones), un groupe phénylalkyle dans lequel le groupe phényle est éventuellement substitué ;

$R_1$ et $R_2$ considérés séparément désignent l'hydrogène, un groupe alkyle droit ou ramifié ayant de 1 à 18 atomes de carbone, un groupe aralkyle, un groupe cycloalkyle ou un groupe phényle éventuellement substitué,

ou encore $R_1$ et $R_2$ considérés ensemble avec l'atome d'azote auquel ils sont liés représentent un hétérocycle à 5 ou 6 chaînons comprenant éventuellement un second hétéroatome tel que la pyrrolidine, la morpholine, la pipérazine ou la pyridine et ses dérivés partiellement ou totalement hydrogénés.

2. Amide selon la revendication 1, caractérisé en ce que R est le groupe $CH_3—(CH_2)_2—$ ; m = n =1 ; X est l'hydrogène et $R_1 = R_2 = (CH_2)_3—CH_3$.

3. Amide selon la revendication 1, caractérisé en ce que R est le groupe $CH_3—(CH_2)_2—$ ; m = n = 1 ; X est l'hydrogène ; $R_1$ est l'hydrogène et $R_2$ est le groupe $—(CH_2)_3—CH_3$.

4. Procédé de préparation des amides selon la revendication 1, caractérisé en ce qu'on utilise comme produit de départ un aminoacide de formule

$$H_2N\text{-}(CH_2)_m\text{-}\overset{X}{CH}\text{-}(CH_2)_n\text{-}COOR'$$

dans laquelle m, n et X ont les significations données dans la revendication 1 et R′ est un atome d'hydrogène ou un groupe alkyle inférieur ($C_1$-$C_4$), que l'on fait réagir ledit produit sur un chlorure d'acide de formule R—COCl de façon à transformer l'amine du produit de départ en un radical amide et que l'on soumet le produit obtenu à une amidification avec un produit de formule

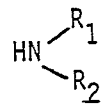

5. Procédé selon la revendication 4, caractérisé en ce que l'amidification est effectuée, par réaction d'un produit dans lequel R′ est alkyle inférieur avec l'amine

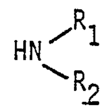

en opérant en solution dans l'alcool ou en utilisant un excès d'amine comme solvant, et à une température comprise entre environ 0 et environ 50 °C.

6. Procédé selon la revendication 5, caractérisé en ce que l'on effectue l'amidification d'un acide (R′ = H) que l'on transforme au préalable en ester d'un alcool inférieur.

7. Procédé selon la revendication 4, caractérisé en ce que l'on effectue l'amidification sur un anhydride mixte de l'acide (R′ = H) par action sur l'acide d'un chloroformiate d'éthyle en présence d'un agent alcalin tel que la triéthylamine.

8. Médicaments nouveaux utiles notamment pour le traitement des affections neurologiques et psychiques, caractérisés en ce qu'ils comportent, en tant que principe actif, au moins un produit selon l'une quelconque des revendications 1 à 3.

9. Médicaments selon la revendication 8, caractérisés en ce qu'ils sont conditionnés en vue d'une administration journalière à l'homme à une dose de 0,1 à 1 g en une ou plusieurs prises.

10. Médicaments selon la revendication 9, caractérisés en ce qu'ils sont conditionnés en vue d'une administration orale.

11. Médicaments selon la revendication 9, caractérisés en ce qu'ils sont conditionnés en vue d'une administration par injection.

**Revendications** (pour l'Etat contractant AT)

1. Procédé de préparation d'amides de l'acide amino-4 butyrique répondant à la formule générale :

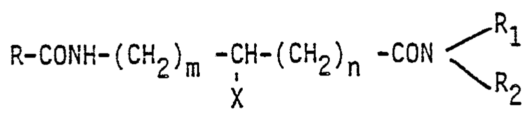

(I)

dans laquelle :

R désigne un groupe alkyle droit ou ramifié ayant de 1 à 7 atomes de carbone ou un groupe cycloalkyle ;

m représente un nombre entier de 0 à 3 ;

n représente un nombre entier de 0 à 2, avec la condition que m + n soit égal ou supérieur à 1 ;

X représente l'hydrogène, un groupe alkyle inférieur (1 à 4 carbones), un groupe phénylalkyle dans lequel le groupe phényle est éventuellement substitué ;

$R_1$ et $R_2$ considérés séparément désignent l'hydrogène, un groupe alkyle droit ou ramifié ayant de 1 à 18 atomes de carbone, un groupe aralkyle, un groupe cycloalkyle ou un groupe phényle éventuellement substitué ;

ou encore $R_1$ et R2 considérés ensemble avec l'atome d'azote auquel ils sont liés représentent un hétérocycle à 5 ou 6 chaînons comprenant éventuellement un second hétéroatome tel que la pyrrolidine, la morpholine, la pipérazine ou la pyridine et ses dérivés partiellement ou totalement hydrogénés, caractérisé en ce qu'on utilise comme produit de départ un aminoacide de formule

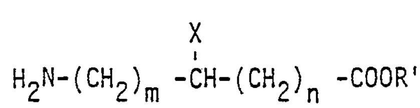

dans laquelle m, n et X sont tels que définis ci-dessus et R′ est un atome d'hydrogène ou un groupe alkyle inférieur ($C_1$-$C_4$), en ce que l'on fait réagir ledit produit sur un chlorure d'acide de formule R—COCl de façon à transformer l'amine du produit de départ en un radical amide, et en ce que l'on soumet le produit obtenu à une amidification avec un produit de formule

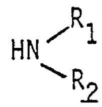

2. Procédé selon la revendication 1, caractérisé en ce que l'amidification est effectuée par réaction d'un produit dans lequel R' est alkyle inférieur avec l'amine

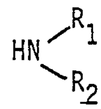

en opérant en solution dans l'alcool ou en utilisant un excès d'amine comme solvant, et à une température comprise entre environ 0 et environ 50 °C.

3. Procédé selon la revendication 2, caractérisé en ce que l'on effectue l'amidification d'un acide (R' = H) que l'on transforme au préalable en ester d'un alcool inférieur.

4. Procédé selon la revendication 1, caractérisé en ce que l'on effectue l'amidification sur un anhydride mixte de l'acide (R' = H) par action sur l'acide d'un chloroformiate d'éthyle en présence d'un agent alcalin tel que la triéthylamine.

5. Procédé selon l'une quelconque des revendications 1 à 4, caractérisé en ce que l'on utilise un aminoacide répondant à la formule 1, dans laquelle m = n = 1 ; X est l'hydrogène ; $R_1 = R_2 = (CH_2)_3-CH_3$, et un chlorure d'acide de formule R—COCl dans laquelle R est le groupe $CH_3-(CH_2)_2$.

6. Procédé selon l'une quelconque des revendications 1 à 4, caractérisé en ce que l'on utilise un aminoacide répondant à la formule 1 dans laquelle m = n = 1 ; X est l'hydrogène ; $R_1$ est l'hydrogène et $R_2$ est le groupe $-(CH_2)_3-CH_3$, et un chlorure d'acide de formule R—COCl dans laquelle R est le groupe $CH_3-(CH_2)_2$.

7. Utilisation des amides obtenus par le procédé selon l'une quelconque des revendications 1 à 6, pour la préparation de médicaments utiles pour le traitement des affections neurologiques et psychiques.

8. Utilisation selon la revendication 7, caractérisée en ce que lesdits médicaments sont conditionnés en vue d'une administration journalière à l'homme à une dose de 0,1 à 1 g en une ou plusieurs prises.

9. Utilisation selon la revendication 7, caractérisée en ce que lesdits médicaments sont conditionnés en vue d'une administration orale.

10. Utilisation selon la revendication 7, caractérisée en ce que lesdits médicaments sont conditionnés en vue d'une administration par injection.

**Claims** (for the contracting states : BE, CH, DE, FR, GB, IT, LI, LU, NL, SE)

1. Amides of 4-aminobutyric acid corresponding to general formula :

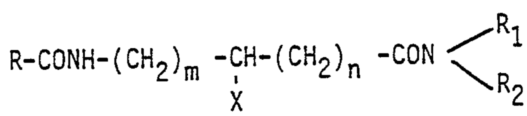

(I)

in which :

R designates a linear or branched alkyl group having from 1 to 7 carbon atoms or a cycloalkyl group ;

m represents an integer of from 0 to 3 ;

n represents an integer of from 0 to 2, on condition that m + n is equal to or greater than 1 ;

X represents hydrogen, a lower alkyl group (1 to 4 carbons), a phenylalkyl group in which the phenyl group is possibility substituted,

$R_1$ and $R_2$ considered separately designate hydrogen, a linear or branched alkyl group having from 1 to 18 carbon atoms, an aralkyl group, a cycloalkyl group or a possibly substituted phenyl group,

or $R_1$ and $R_2$ considered together with the nitrogen atom to which they are attached represent a heterocycle with 5 or 6 groupings possibly comprising a second heteroatom such as pyrrolidine, morpholine, piperazine or pyridine and its partially or totally hydrogenated derivatives.

2. Amide according to claim 1, characterized in that R is the $CH_3-(CH_2)_2-$ group ; m = n =1 ; X is hydrogen and $R_1 = R_2 = (CH_2)_3-CH_3$.

3. Amide according to claim 1, characterized in that R is the $CH_3-(CH_2)_2-$m = n = 1 ; X is hydrogen $R_1$ is hydrogen and $R_2$ is the $-(CH_2)_3-CH_3$ group.

4. A process for preparing the amides according to claim 1, characterized in that the starting product is constituted by an aminoacid of formula :

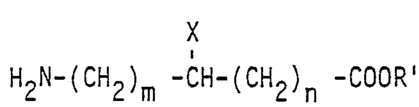

in which m, n and X have the meanings given in claim 1 and R' is a hydrogen atom or a lower alkyl group (1 to 4 carbon atoms), said product is reacted on an acid chloride of formula R COCl so as to convert the amine of the starting product into an amide radical and the product obtained is subjected to an amidification with a product of formula

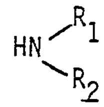

5. The process according to claim 4, characterized in that amidification is effected by reaction of a product in which R' is lower alkyl with the amine

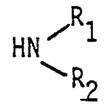

operating in solution in alcohol or using an excess of amine as solvent, and at a temperature of between about 0 and about 50 °C.

6. The process according to claim 5, characterized in that amidification of an acid (R' = H) which is previously converted into ester of a lower alcohol is effected.

7. The process according to claim 1, characterized in that amidification is effected on a mixed anhydride of the acid (R' = H) by action on the acid of an ethyl chloroformate in the presence of an alkaline agent such as triethylamine.

8. New drugs particularly useful for the treatment of neurological and psychic disorders, characterized in that they comprise, as active ingredient, at least on product according to any one of claims 1 to 3.

9. The drugs according to claim 8, characterized in that they are prepared with a view to daily human administration at a dose of 0.1 to 1g in one or several doses.

10. The drugs according to claim 9, characterized in that they are prepared with a view to oral administration.

11. The drugs according to claim 9, characterized in that they are prepared with a view to administration by injection.

**Claims** (for the contracting state AT)

1. Process for preparing amides of 4-aminobutyric acid corresponding to general formula :

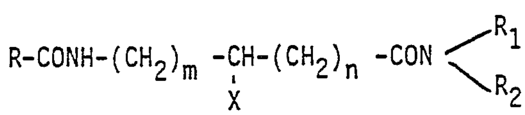
(I)

in which :

R designates a linear or branched alkyl group having from 1 to 7 carbon atoms or a cycloalkyl group ;

m represents an integer of from 0 to 3 ;

n represents an integer from 0 to 2, condition that m + n is equal to or greater than 1 ;

X represents hydrogen, a lower alkyl group (1 to 4 carbons), a phenylalkyl group in which the phenyl group is possibly substituted,

$R_1$ and $R_2$ considered separately designate hydrogen, a linear or branched alkyl group having from 1 to 18 carbon atoms, an aralkyl group, a cycloalkyl group or a possibly substituted phenyl group,

or $R_1$ an $R_2$ considered together with the nitrogen atom to which they are attached represent a theterocycle with 5 or 6 groupings possibly comprising a second heteroatom such as pyrrolidine, morpholine, piperazine or pyridine and its partially or totally hydrogenated derivatives, characterized in that the starting product is constituted by an aminoacid of formula :

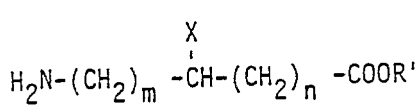

in which m, n and X are such as defined above and R' is a hydrogen atom or a lower alkyl group (1 to 4 carbon atoms), said product is reacted on an acid chloride of formula R—COCl so as to convert the amine of the starting product into an amide radical and the product obtained is subjected to an amidification with a product of formula

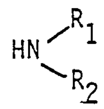

2. The process according to claim 1, characterized in that amidification is effected by reaction of a product in which R' is lower alkyl with the amine,

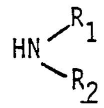

operating in solution in alcohol or using an excess of amine as solvent, and at a temperature of between about 0 and about 50 °C.

3. The process according to claim 2, characterized in that amidification of an acid (R' = H) which is previously converted into ester of a lower alcohol is effected.

4. The process according to claim 1, characterized in that amidification is effected on a mixed anhydride of the acid (R' = H) by action on the acid of an ethyl chloroformate in the presence of an alkaline agent such as triethylamine.

5. The process according to any one of claims 1 to 4, characterized in that an aminoacid responding to formula 1 is used, in which m = n = 1 ; X is hydrogen ; $R_1 = R_2 = (CH_2)_3 — CH_3$, and an acid chloride of formula R—COCl in which R is the $CH_3 — (CH_2)_2$— group.

6. The process according to any one of claims 1 to 4, characterized in that an aminoacid responding to formula I in which m = n = 1 ; X is hydrogen ; $R_1$ is hydrogen and $R_2$ is the $—(CH_2)_3—CH_3$ group, and an acid chloride of formula R—COCl in which R is the $CH_3—(CH_2)_2$— group.

7. Use of amides obtained by the process according to any one of claims 1 to 6, for the preparation of drugs useful for the treatment of neurological and psychic disorders.

8. Use according to claim 7, characterized in that said drugs are prepared with a view to daily administration human at a dose of 0.1 to 1 g in one or several doses.

9. Use according to claim 7, characterized in that said drugs are prepared with a view to oral administration.

10. Use according to claim 7, characterized in that said drugs are prepared with a view to administration by injection.

**Patentansprüche** (für die Vertragsstaaten : BE, CH, DE, FR, GB, IT, LI, LU, NL, SE)

1. 4-Amino-buttersäureamide der allgemeinen Formel

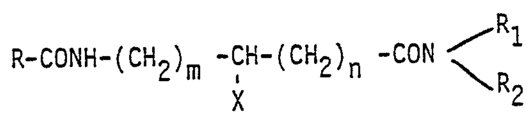

(I)

worin

R eine gerade oder verzweigte Alkylgruppe mit 1 bis 7 kohlenstoffatomen oder eine Cycloalkylgruppe bedeutet ;

m für eine ganze Zahl von 0 bis 3 steht ;

n für eine ganze Zahl von 0 bis 2 steht, mit der Maßgabe, daß m + n gleich oder größer als 1 sind ;

X Wasserstoff, eine Niedrigalkylgruppe (1 bis 4 Kohlenstoffatome) oder eine Phenylalkylgruppe, in der die Phenylgruppe gegebenenfalls substituiert ist, darstellt ;

$R_1$ und $R_2$, getrennt betrachtet, Wasserstoff, eine gerade oder verzweigte Alkylgruppe mit 1 bis 18 Kohlenstoffatomen, eine Aralkylgruppe, eine Cycloalkylgruppe oder eine gegebenenfalls substituierte Phenylgruppe darstellen ;

oder aber $R_1$ und $R_2$, zusammen mit dem Stickstoffatom, an das sie gebunden sind, betrachtet, einen 5- oder 6-gliedrigen, gegebenenfalls ein zweites Heteroatom enthaltenden Heterocyclus, wie Pyrrolidin, Morpholin, Piperazin oder Pyridin und deren teilweise oder vollständig hydrierten Derivate, bedeuten.

2. Amid nach Anspruch 1, dadurch gekennzeichnet, daß R für die Gruppe $CH_3—(CH_2)_2$— steht ; m = n = 1 ; X Wasserstoff bedeutet und $R_1 = R_2 = (CH_2)_3—CH_3$.

3. Amid nach Anspruch 1, dadurch gekennzeichnet, daß R für die Gruppe $CH_3—(CH_2)_2$— steht ; m = n = 1 ; X Wasserstoff bedeutet ; $R_1$ Wasserstoff bedeutet und $R_2$ die Gruppe $—(CH_2)_3—CH_3$ darstellt.

4. Verfahren zur Herstellung der Amide nach Anspruch 1, dadurch gekennzeichnet, daß man als Ausgangsprodukt eine Aminosäure der Formel

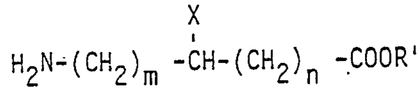

verwendet, worin m, n und X die in Anspruch 1 angegebene Bedeutung haben und R' für ein Wasserstoffatom oder eine Niedrigalkylgruppe ($C_1$-$C_4$) steht, daß man das Produkt mit einem Säurechlorid der Formel R—COCl reagieren läßt, sodaß die Aminofunktion des Ausgangsprodukts in einen Amidrest umgewandelt wird, und daß man das erhaltene Produkt einer Amidierung mit einem Produkt der Formel

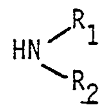

unterzieht.

5. Verfahren nach Anspruch 4, dadurch gekennzeichnet, daß die Amidierung durch Umsetzung eines Produkts, in welchem R' Niedrigalkyl bedeutet, mit dem Amin

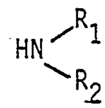

durchgeführt wird, wobei in Lösung in Alkohol oder unter Verwendung eines Aminüberschusses als Lösungsmittel und bei einer Temperatur zwischen etwa 0 und etwa 50 °C gearbeitet wird.

6. Verfahren nach Anspruch 5, dadurch gekennzeichnet, daß die Amidierung einer Säure (R' = H), die man zuvor in den Ester eines Niedrigalkohols überführt, durchgeführt wird.

7. Verfahren nach Anspruch 4, dadurch gekennzeichnet, daß die Amidierung mit einem gemischten Anhydrid der Säure (R' = H) durch Einwirken eines Äthylchlorformiats in Gegenwart eines alkalischen Mittels, wie Triäthylamin, auf die Säure durchgeführt wird.

8. Neue Medikamente, die insbesondere zur Behandlung von neurologischen und psychischen Erkrankungen nützlich sind, dadurch gekennzeichnet, daß sie als Wirkstoff mindestens ein Produkt nach einem der Ansprüche 1 bis 3 enthalten.

9. Medikamente nach Anspruch 8, dadurch gekennzeichnet, daß sie für eine tägliche Verabreichung in einer Dosis von 0,1 bis 1 g in einer oder mehreren Einnahmen an den Menschen konditioniert sind.

10. Medikamente nach Anspruch 9, dadurch gekennzeichnet, daß sie für orale Verabreichung konditioniert sind.

11. Medikamente nach Anspruch 9, dadurch gekennzeichnet, daß sie für Verabreichung durch Injektion konditioniert sind.

**Patentansprüche** (für den Vertragsstaat AT)

1. Verfahren zur Herstellung von 4-Amino-buttersäureamiden der allgemeinen Formel

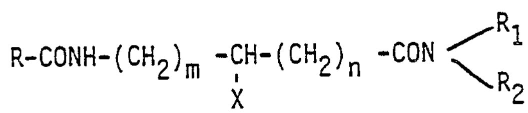

worin :

R eine gerade oder verzweigte Alkylgruppe mit 1 bis 7 Kohlenstoffatomen oder eine Cycloalkylgruppe bedeutet ;

m für eine ganze Zahl von 0 bis 3 steht ;

n für eine ganze Zahl von 0 bis 2 steht, mit der Maßgabe, daß m + n gleich oder größer als 1 sind ;

X Wasserstoff, eine Niedrigalkylgruppe (1 bis 4 Kohlenstoffatome) oder eine Phenylalkylgruppe, in der die Phenylgruppe gegebenenfalls substituiert ist, darstellt ;

$R_1$ und $R_2$, getrennt betrachtet, Wasserstoff, eine gerade oder verzweigte Alkylgruppe mit 1 bis 18 Kohlenstoffatomen, eine Aralkylgruppe, eine Cycloalkylgruppe oder eine gegebenenfalls substituierte Phenylgruppe darstellen ;

oder aber $R_1$ und $R_2$, zusammen mit dem Stickstoffatom, an das sie gebunden sind, betrachtet, einen 5- oder 6-gliedrigen, gegebenenfalls ein zweites Heteroatom enthaltenden Heterocyclus, wie Pyrrolidin, Morpholin, Piperazin oder Pyridin und deren teilweise oder vollständig hydrierten Derivate, bedeuten, dadurch gekennzeichnet, daß man als Ausgangsprodukt eine Aminosäure der Formel

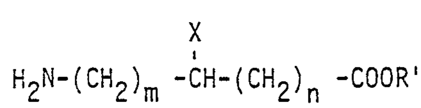

verwendet, worin m, n und X die oben angegebene Bedeutung haben und R' für ein Wasserstoffatom oder eine Niedrigalkylgruppe ($C_1-C_4$) steht, daß man das Produkt mit einem Säurechlorid der Formel R—COCL reagieren läßt, sodaß die Aminofunktion des Ausgangsprodukts in einen Amidrest umgewandelt wird, und daß man das erhaltene Produkt einer Amidierung mit einem Produkt der Formel unterzieht

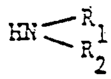

2. Verfahren nach Anspruch 1, dadurch gekennzeichnet, daß die Amidierung durch Umsetzung eines Produkts, in welchem R' Niedrigalkyl bedeutet, mit dem Amin

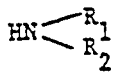

durchgeführt wird, wobei in Lösung in Alkohol oder unter Verwendung eines Aminüberschusses als Lösungsmittel und bei einer Temperatur zwischen etwa 0 und etwa 50 °C gearbeitet wird.

3. Verfahren nach Anspruch 2, dadurch gekennzeichnet, daß die Amidierung einer Säure (R' = H), die man zuvor in den Ester eines Niedrigalkohols überführt, durchgeführt wird.

4. Verfahren nach Anspruch 1, dadurch gekennzeichnet, daß die Amidierung mit einem gemischten Anhydrid der Säure (R' = H) durch Einwirken eines Äthylchlorformiats in Gegenwart eines alkalischen Mittels, wie Triäthylamin, auf die Säure durchgeführt wird.

5. Verfahren nach einem der Ansprüche 1 bis 4, dadurch gekennzeichnet, daß man eine Aminosäure der Formel (1), worin m = n = 1, X Wasserstoff bedeutet und $R_1 = R_2 = (CH_2)_3—CH_3$, und ein Säurechlorid der Formel R—COCl, worin R die Gruppe $CH_3—(CH_2)_2—$ darstellt, verwendet.

6. Verfahren nach einem der Ansprüche 1 bis 4, dadurch gekennzeichnet, daß man eine Aminosäure der Formel (1), worin m = n = 1, X Wasserstoff bedeutet, $R_1$ für Wasserstoff steht und $R_2$ die Gruppe $—(CH_2)_3—CH_3$ darstellt, und ein Säurechlorid der Formel R—COCL, worin R die Gruppe $CH_3—(CH_2)_2—$ darstellt, verwendet.

7. Verwendung der durch das Verfahren nach einem der Ansprüche 1 bis 6 erhaltenen Amide zur Herstellung von Medikamenten, die zur Behandlung von neurologischen und psychischen Erkrankungen nützlich sind.

8. Verwendung nach Anspruch 7, dadurch gekennzeichnet, daß die Medikamente für eine tägliche Verabreichung in einer Dosis von 0,1 bis 1 g in einer oder mehreren Einnahmen an den Menschen konditioniert sind.

9. Verwendung nach Anspruch 7, dadurch gekennzeichnet, daß die Medikamente für orale Verabreichung konditioniert sind.

10. Verwendung nach Anspruch 7, dadurch gekennzeichnet, daß die Medikamente für Verabreichung durch Injektion konditioniert sind.